# EUROPÄISCHE PATENTSCHRIFT

(11) **EP 3 162 319 B1**
(45) Veröffentlichungstag und Bekanntmachung des Hinweises auf die Patenterteilung: **28.02.2018**
(21) Anmeldenummer: 15192143.4
(22) Anmeldetag: 29.10.2015
(51) Int. Cl.: A61C 1/00, G06F 19/00, A61G 15/14

(54) **ZAHNÄRZTLICHES BEHANDLUNGS- ODER UNTERSUCHUNGSSYSTEM SOWIE VERFAHREN ZUM BETREIBEN EINES SOLCHEN**
DENTAL TREATMENT OR INSPECTION SYSTEM AND METHOD FOR OPERATING SAME
SYSTÈME D'ANALYSE OU DE TRAITEMENT DENTAIRE ET SON PROCÉDÉ DE FONCTIONNEMENT

(43) Veröffentlichungstag der Anmeldung: 03.05.2017
(73) Patentinhaber: Kaltenbach & Voigt GmbH, 88400 Biberach (DE)
(72) Erfinder: Bürk, Richard, 88433 Alberweiler (DE); Sauter, Johannes, 88416 Mittelbruch (DE)
(74) Vertreter: Thun, Clemens

(56) Entgegenhaltungen:
- EP-A1- 0 017 318
- WO-A1-2010/051080
- DE-A1- 2 712 734
- DE-A1- 4 409 862
- DE-A1- 10 139 020
- DE-A1- 19 508 481
- DE-A1-102008 025 543
- DE-C1- 19 702 996

## Beschreibung

Die Erfindung betrifft ein zahnärztliches Behandlungs- oder Untersuchungssystem sowie ein Verfahren zum Betreiben eines solchen.

Ein zahnärztliches Behandlungs- oder Untersuchungssystem ist aus dem Stand der Technik in Form einer zahnärztlichen Behandlungseinheit bekannt. Eine solche zahnärztliche Behandlungseinheit gemäß dem Stand der Technik umfasst ein so genanntes Arztelement mit mehreren köcherartigen Instrumentenhaltern für dentale Instrumente. Zur Erkennung einer Entnahme eines dieser Instrumente aus seinem Instrumentenhalter bzw. zur Selektion eines dieser Instrumente zur Initialisierung seiner Ansteuerung dient ein im Bereich des Instrumentenhalters angeordneter Mikroschalter, ein Reedkontakt oder ein Druckschalter oder ein separater Vorwahlschalter. Die Integration eines derartigen Mikroschalters, Reedkontakts oder Druckschalters in die Behandlungseinheit ist mit einem vergleichsweise hohen Aufwand verbunden. Ein separater Vorwahlschalter erfordert eine vergleichsweise unkomfortable Bedienung.

Wenn für eines der Instrumente ein Parameter, beispielsweise eine Motordrehzahl, programmiert werden soll, muss hierzu das betreffende Instrument zunächst selektiert bzw. vorgewählt werden, entweder durch eine Entnahme des Instruments aus seinem Instrumentenhalter oder mittels einem, auf einem Display angezeigten Menü. Beides erfordert einen erhöhten Bedienungsaufwand.

Aus der DE 197 02 996 C1 ist eine Einrichtung zur Erkennung der Entnahme eines an einen Versorgungsschlauch angeschlossenen zahnärztlichen Handstücks aus einer Ablagevorrichtung bekannt.

Aus der EP 0 017 318 A1 ist ein zahnärztliches Behandlungssystem bekannt, bei dem ein Berühren eines Handgeräts durch Erfassen einer Kapazitätsänderung erkannt wird. Bei der Berührung wird eine Lichtquelle eingeschaltet und das so erzeugte Licht dem Handgerät über einen Lichtleiter zugeführt.

DE2712734 offenbart eine Anordnung zur Steuerung der elektrischen Leistung zahnärztlicher Handinstrumente. Der Erfindung liegt die Aufgabe zugrunde, ein entsprechendes verbessertes zahnärztliches Behandlungs- oder Untersuchungssystem anzugeben; insbesondere soll das System bei einfacher Herstellungsmöglichkeit einen verbesserten Bedienkomfort bieten. Außerdem soll ein entsprechendes Verfahren zum Betreiben eines zahnärztliches Behandlungs- oder Untersuchungssystem angegeben werden.

Diese Aufgabe wird gemäß der Erfindung mit den in den unabhängigen Ansprüchen genannten Gegenständen gelöst. Besondere Ausführungsarten der Erfindung sind in den abhängigen Ansprüchen angegeben.

Gemäß der Erfindung ist ein zahnärztliches Behandlungs- oder Untersuchungssystem vorgesehen, das mehrere Behandlungs- oder Untersuchungsinstrumente umfasst, sowie eine mit den mehreren Behandlungs- oder Untersuchungsinstrumenten gekoppelte Versorgungseinheit. Dabei weist das System Mittel zum Erkennen des Berührens eines ausgewählten Instruments von den mehreren Behandlungs- oder Untersuchungsinstrumenten auf, wobei abhängig von einer erkannten Berührung des ausgewählten Behandlungs- oder Untersuchungsinstruments die Versorgungseinheit für einen Betrieb des ausgewählten Behandlungs- oder Untersuchungsinstruments initialisiert wird. Dieses Initialisieren kann beispielsweise das zur Verfügung stellen von Ansteuer- oder Parametrieroptionen für das ausgewählte Behandlungs- oder Untersuchungsinstrument und/oder von Medien wie z. B. Luft oder Wasser umfassen. Auch das elektrische Verbinden des ausgewählten Behandlungs- oder Untersuchungsinstruments mit einem Leistungsausgang einer Steuereinheit kann ein Initialisieren im Sinne der Erfindung darstellen.

Durch die Mittel zum Erkennen des Berührens lässt sich somit erzielen, dass die Versorgungseinheit beispielsweise die Ansteuer- oder Parametrieroptionen für das betreffende, also das durch des Berühren ausgewählte Behandlungs- oder Untersuchungsinstrument und/oder die geeigneten Medien automatisch zur Verfügung stellt, ohne dass hierzu eine weitergehende Manipulation seitens des Benutzers des Behandlungs- oder Untersuchungssystems erforderlich wäre. Hierdurch ist die Bedienung erleichtert.

Erfindungsgemäß umfassen dabei die Mittel zum Erkennen des Berührens mindestens einen kapazitiven Sensor. Ein kapazitiver Sensor eignet sich besonders zur Berührungserkennung. Zudem lässt sich auf diese Weise erzielen, dass hierbei kein entsprechender Mikroschalter, Reedkontakt oder Druckschalter oder dergleichen erforderlich ist. Dies ist insbesondere mit Bezug auf eine erleichterte und kostengünstige Herstellungsmöglichkeit des Systems vorteilhaft.

Vorzugsweise ist das Behandlungs- oder Untersuchungsinstruments dabei derart gestaltet, dass die Mittel zum Erkennen des Berührens die kapazitive Einkopplung auf das Gehäuse des berührten bzw. ausgewählten Behandlungs- oder Untersuchungsinstruments - bzw. kurz Instruments - oder auf Leitungen davon bewerten, wobei die kapazitive Einkopplung insbesondere durch Messen von Brumm- oder Störspannungen ermittelt wird. Alternativ hierzu kann die Gestaltung derart sein, dass die Mittel zum Erkennen des Berührens eine Kapazitätsänderung des Behandlungs- oder Untersuchungsinstruments bewerten.

Vorzugsweise umfassen die Mittel zum Erkennen des Berührens eine in der Versorgungseinheit angeordnete Bewertungseinheit, welche über eine Sensorleitung mit den Behandlungs- oder Untersuchungsinstrumenten verbunden ist. Dabei ist weiterhin vorzugsweise die Sensorleitung durch eine Stromversorgungsleitung für die Behandlungs- oder Untersuchungsinstrumente gebildet.

Das Mittel zum Erkennen der Berührung eines Behandlungs- oder Untersuchungsinstruments kann gegebenenfalls auch in das Instrument integriert sein.

Erfindungsgemäß ist das Behandlungs- oder Untersuchungssystem derart gestaltet, dass ein Erkennen des Berührens des ausgewählten Behandlungs- oder Untersuchungsinstruments lediglich in einem nicht aktivierten Zustand der zur Auswahl stehenden Behandlungs- oder Untersuchungsinstrumente durchgeführt wird. Auf diese Weise lässt sich die Gefahr verringern, dass während eines Arbeitens mit dem Instrument die Medienzufuhr unterbrochen wird.

Erfindungsgemäß ist die Gestaltung weiterhin derart, dass die Versorgungseinheit ein Display aufweist oder mit einem Display verbunden, auf dem eine Darstellung von Betriebsparametern des ausgewählten Behandlungs- oder Untersuchungsinstruments erfolgt, wobei die Darstellung abhängig von dem Erkennen des Berührens des ausgewählten Behandlungs- oder Untersuchungsinstruments erfolgt. Auf diese Weise lässt sich vermeiden, dass zur Einstellung von Betriebsparametern zunächst durch eine anderweitige Manipulation, beispielsweise durch Manipulation eines Vorwahlschalters, das betreffende Behandlungs- oder Untersuchungsinstruments ausgewählt bzw. selektiert werden muss.

Gemäß einem weiteren Aspekt der Erfindung ist ein Verfahren zum Betreiben eines zahnärztlichen Behandlungs- oder Untersuchungssystems mit mehreren Behandlungs- oder Untersuchungsinstrumenten sowie mit einer mit den mehreren Behandlungs- oder Untersuchungsinstrumenten gekoppelten Versorgungseinheit vorgesehen. Dabei wird ein Berühren der Behandlungs- oder Untersuchungsinstrumente überwacht und abhängig von einer erkannten Berührung eines der Behandlungs- oder Untersuchungsinstrumente die Versorgungseinheit für einen Betrieb des ausgewählten Behandlungs- oder Untersuchungsinstruments initialisiert. Hierdurch lässt sich erzielen, dass die Selektion, also das Auswählen des betreffenden Instruments allein durch das Berühren von diesem Instrument erfolgt. Die Bedienung des Systems ist auf diese Weise erleichtert.

Das erfindungsgemäße Initialisieren kann beispielsweise das zur Verfügung stellen von Ansteuer- oder Parametrieroptionen für das ausgewählte Behandlungs- oder Untersuchungsinstrument und/oder von Medien wie z. B. Luft oder Wasser umfassen. Auch das elektrische Verbinden des ausgewählten Behandlungs- oder Untersuchungsinstruments mit einem Leistungsausgang einer Steuereinheit kann gemäß der Erfindung im Rahmen der Initialisierung erfolgen.

Erfindungsgemäß wird dabei zum Erkennen des Berührens des einen der Behandlungs- oder Untersuchungsinstrumente mindestens ein kapazitiver Sensor verwendet.

Weiterhin vorzugsweise wird dabei zum Erkennen des Berührens die kapazitive Einkopplung auf das Gehäuse des betreffenden Behandlungs- oder Untersuchungsinstruments oder auf Leitungen davon bewertet, wobei die kapazitive Einkopplung insbesondere durch Messen von Brumm- oder Störspannungen ermittelt wird. Alternativ wird zum Erkennen des Berührens eine Kapazitätsänderung des betreffenden Behandlungs- oder Untersuchungsinstruments bewertet.

Erfindungsgemäß wird ein Erkennen des Berührens des einen der Behandlungs- oder Untersuchungsinstrumente lediglich in einem nicht aktivierten Zustand des betreffenden Behandlungs- oder Untersuchungsinstruments durchgeführt.

Erfindungsgemäß weist die Versorgungseinheit ein Display auf oder ist mit einem Display verbunden, auf dem eine Darstellung von Betriebsparametern des einen der Behandlungs- oder Untersuchungsinstrumente erfolgt, wobei die Darstellung abhängig von dem Erkennen des Berührens des betreffenden Behandlungs- oder Untersuchungsinstruments erfolgt.

Die Erfindung wird im Folgenden anhand eines Ausführungsbeispiels und mit Bezug auf die Zeichnungen näher erläutert. Es zeigen
Fig. 1 eine schematische Prinzip-Skizze zu einem erfindungsgemäßen zahnärztlichen Behandlungs- oder Untersuchungssystem,
Fig. 2a eine Skizze zu einer möglichen Ausführungsform der Mittel zum Erkennen des Berührens,
Fig. 2b eine Skizze zu einer weiteren möglichen Ausführungsform der Mittel zum Erkennen des Berührens,
Fig. 3a eine Skizze zu einer möglichen Ausführungsform des Instruments,
Figuren 3b bis 3d Skizzen zu weiteren möglichen Ausführungsformen des Instruments,
Figuren 4a, 4b Skizzen zu einer möglichen Darstellung auf dem Display und
Fig. 5 eine Skizze zu einer möglichen Ausführung der Schaltmatrix.

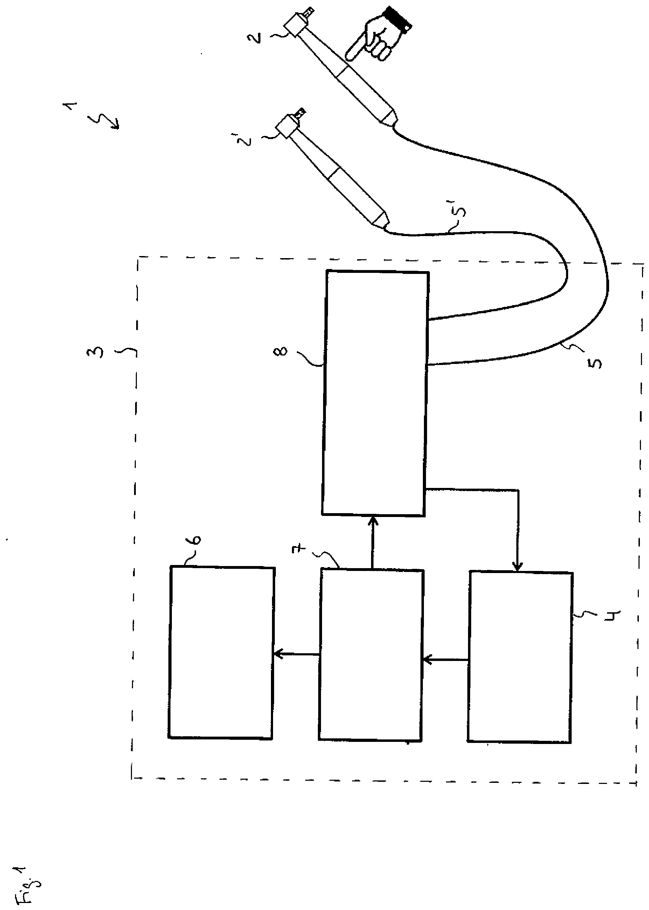

In Fig. 1 ist eine schematische Skizze zu einem erfindungsgemäßen zahnärztlichen Behandlungs- oder Untersuchungssystem 1, im Folgenden auch kurz als System 1 bezeichnet, dargestellt. Das System 1 kann insbesondere in Form einer dentalen Behandlungseinheit gegeben sein. Das System 1 weist mehrere Behandlungs- oder Untersuchungsinstrumente 2, 2' auf, im Folgenden auch kurz als Instrumente 2, 2' bezeichnet. Das System 1 kann (in den Figuren nicht gezeigte) Instrumentenhalter für die Instrumente 2, 2' aufweisen, die für eine Lagerung der Instrumente 2, 2' in einem Zeitraum vorgesehen ist, in dem die Instrumente 2, 2' nicht benutzt werden. Der Instrumentenhalter kann beispielsweise an einem so genannten Arztelement der Behandlungseinheit gebildet sein. Der Instrumentenhalter ist dabei derart gestaltet, dass sich die Instrumente 2 durch einen Nutzer des Systems 1, also beispielsweise einen Zahnarzt, leicht, lediglich unter Nutzung einer Hand, entnommen werden können.

Die Instrumente 2, 2' können beispielsweise ein dentales Elektromotor-Handstück umfassen oder eine dentale Turbine, eine dentale Kamera, ein Ultraschall-Gerät oder ein druckluftbetriebenes Instrument etc.

Weiterhin umfasst das System 1 eine Versorgungseinheit 3, die mit den Instrumenten 2, 2' gekoppelt ist und die insbesondere dazu ausgebildet ist, die Instrumente 2, 2' mit Medien wie Luft und/oder Wasser und/oder Strom zu versorgen, insbesondere über Versorgungsleitungen 5, 5', die mit den Instrumenten 2, 2' verbunden sind. Außerdem ist die Versorgungseinheit 3 vorzugsweise dazu ausgebildet, zur Bedienung der Instrumente 2, 2' Ansteuer- oder Parametrieroptionen für die Instrumente 2, 2' zur Verfügung zu stellen. Bei den Ansteuer- und Parametrieroptionen kann es sich beispielsweise um Einstellparameter handeln, wie beispielsweise eine Motordrehzahl im Fall eines Elektromotor-Handstücks. Auch das zur Verfügung stellen entsprechender Ansteuermöglichkeiten wir z.B. das Einstellen einer Motordrehzahl mit Hilfe eines sog. Fußanlassers stellt ein Initialisieren der Versorgungseinheit im Sinne der Erfindung dar.

Weiterhin umfasst das System 1 Mittel 4 zum Erkennen eines Berührens der Instrumente 2, 2', insbesondere eines Berührens durch eine menschliche Hand. Dabei ist das System 1 derart gestaltet, dass die Versorgungseinheit 3 abhängig von einer durch die Mittel 4 zum Erkennen erkannten Berührung eines ausgewählten Instruments 2 automatisch für einen Betrieb des ausgewählten Instruments initialisiert wird und hierbei z.B. die Ansteuer- oder Parametrieroptionen und/oder die Medien für das ausgewählte Instrument 2 zur Verfügung stellt. Insbesondere kann die Gestaltung derart sein, dass die Versorgungseinheit 3 die Ansteuer- oder Parametrieroptionen bzw. die Medien für das betreffende ausgewählte Instrument 2 zur Verfügung stellt, sobald durch die Mittel 4 zum Erkennen eine Berührung des betreffenden Instruments 2 erkannt worden ist. Ein Nutzer des Systems 1 kann also eines von den Instrumenten 2, 2' auswählen, indem er es lediglich berührt; das System 1 ist derart gestaltet, dass durch die Mittel 4 zum Erkennen ein solches Berühren von einem der Instrumente 2, 2' erkannt wird und als Folge hiervon von der Versorgungseinheit 3 die Ansteuer- oder Parametrieroptionen und/oder die Medien für das betreffende, also durch das Berühren ausgewählte Instrument 2 zur Verfügung gestellt werden.

Auf diese Weise lässt sich erzielen, dass das ausgewählte Instrument 2 - im Folgenden auch kurz als "das" Instrument 2 bezeichnet - durch die Versorgungseinheit 3 entsprechend mit den Medien versorgt wird bzw. die Ansteuer- oder Parametrieroptionen zur Verfügung gestellt werden, sobald ein Nutzer des Systems 1, also beispielsweise der Zahnarzt, das betreffende Instrument 2 berührt, also beispielsweise aus dem entsprechenden Instrumentenhalter entnimmt, um mit dem Instrument 2 zu arbeiten. Somit erfolgt quasi automatisch eine entsprechende Selektion des Instruments 2 und darauf abgestimmte Initialisierung der Versorgungseinheit 3 für einen Betrieb des Instruments 2. Eine darüber hinausgehende Manipulation des Systems 1 durch den Nutzer ist zur Selektion des Instruments 2 nicht erforderlich. Hierdurch ist insbesondere eine besonders intuitive Bedienung des Systems 1 durch den Nutzer ermöglicht.

Im Folgenden sollen die Maßnahmen zum Erkennen des Berührens eines Instruments näher erläutert werden, wobei offensichtlich diese Maßnahmen dann bei mehreren der Instrumente, idealerweise bei allen Instrumenten des Behandlungsplatzes verwirklicht sind. Erfindungsgemäss umfassen die Mittel 4 zum Erkennen des Berührens einen kapazitiven Sensor. Dieser eignet sich besonders gut dazu, eine Berührung des Instruments 2 zu erfassen. Insbesondere lässt sich auf diese Weise ein entsprechender elektromagnetischer Schalter, wie er aus dem eingangs genannten Stand der Technik bekannt ist, vermeiden, so dass demgegenüber eine besonders kostengünstige Herstellung ermöglicht ist.

Beispielsweise kann dabei weiterhin die Gestaltung derart sein, dass die Mittel 4 zum Erkennen des Berührens die kapazitive Einkopplung auf ein Gehäuse des Instruments 2 oder auf Leitungen davon bewerten, wobei die kapazitive Einkopplung insbesondere durch Messen von Brumm- oder Störspannungen ermittelt wird, die bei Berührung deutlich ansteigen. Alternativ hierzu kann die Gestaltung derart sein, dass die Mittel 4 zum Erkennen des Berührens eine Kapazitätsänderung des Instruments 2 bewerten.

Für die Methode der Kapazitätsmessung können im Allgemeinen geeignete bekannte Methoden wie relaxations Oszillatoren, Ladungstransfermethoden, Ladungs-Zeitmessung o. ä. verwendet werden. Bevorzugt wird jedoch die Methode der Messung der Eigenkapazitätsänderung. Denkbar wäre auch die Messung der Koppelkapazität, sofern zwei entsprechende Leitungen gegeben sind.

Eine besonders geeignete kapazitive Erkennung ist ermöglicht, wenn die Mittel 4 zum Erkennen des Berührens eine in der Versorgungseinheit 3 angeordnete Bewertungseinheit umfassen, welche über eine Sensorleitung mit dem Instrument 2 verbunden ist. Die Sensorleitung kann beispielsweise durch eine eigens hierfür konzipierte Leitung gegeben sein; vorteilhaft ist sie jedoch durch eine als solche ohnehin vorhandene Stromversorgungsleitung für das Instrument 2 gebildet, also beispielsweise durch die oben genannte Versorgungsleitung 5. Hierbei kann es sich beispielsweise um eine Motorphasen-Leitung oder um eine Leitung zur Stromversorgung einer Lichtquelle des Instruments 2 handeln.

Im gezeigten Beispiel weist das System 1 weiterhin eine Schaltmatrix 8 auf, die mit den Versorgungsleitungen 5, 5' und auch mit den Mitteln 4 zum Erkennen des Berührens verbunden ist. Dabei können die Mittel 4 zum Erkennen des Berührens bzw. kann die kapazitive Instrumentenerkennung direkt in der Schaltmatrix 8 an diejenige der Versorgungsleitungen 5 gekoppelt sein, die mit dem ausgewählten Instrument 2 verbunden ist. Sobald durch die Mittel 4 eine Berührung des Instruments 2 erkannt wird, wird dies von den Mitteln 4 an eine Steuereinheit 7 des Systems 1 weitergeleitet. Durch die Steuereinheit 7 wird im Fall einer Erkennung einer Berührung veranlasst, dass die Ansteuer- oder Parametrieroptionen bzw. die Medien für das Instrument 2 zur Verfügung gestellt werden, sobald die Berührung erkannt worden ist. Das zur Verfügung stellen geeigneter Medien kann dabei insbesondere auch das zur Verfügung stellen eines geeigneten Versorgungstrom bzw. das elektrische Verbinden des ausgewählten Instruments mit einem Leistungs- oder Stromversorgungsausgang der Steuereinheit 7 umfassen. Erfindungsgemäss weist die Versorgungseinheit 3 ein Display 6 auf oder ist mit einem Display verbunden, auf dem eine Darstellung von Betriebsparametern des Instruments 2 erfolgt, wobei die Darstellung abhängig von dem Erkennen des Berührens des Instruments 2 erfolgt. Dabei ist die Gestaltung des Systems 1 weiterhin vorzugsweise derart, dass das Display 6 als Bedienoberfläche zur Einstellung von Betriebs-Parametern des Instruments 2 ausgestaltet ist, wobei insbesondere auf dem Display 6 die Ansteuer- oder Parametrieroptionen angezeigt werden bzw. werden können. Erfindungsgemäss erfolgt hierbei während des Motorlaufs des Instruments 2 keine Berührungsdetektion. Allgemeiner formuliert wird vorzugsweise ein Erkennen des Berührens des Instruments 2 lediglich in einem nicht aktivierten Zustand des Instruments 2 durchgeführt. Dies ist insbesondere vorteilhaft, weil hierdurch sichergestellt wird, dass während eines Arbeitens mit dem Instrument 2 die Medienzufuhr nicht unterbrochen wird.

Im gezeigten Beispiel ist die Gestaltung derart, dass durch die Steuereinheit 7 entsprechend der jeweiligen Anwendung die gewünschten Informationen, also beispielsweise die Parametrieroptionen für das Instrument 2 an dem Display 6 angezeigt werden. Erfolgt dann beispielsweise im Fall eines Elektromotor-Handstücks über einen (in den Figuren nicht gezeigten) so genannten Fußanlasser ein Startkommando für das Instrument 2, wird über die Schaltmatrix 8 der Motor des Instruments 2 mit einer Endstufe verbunden und gestartet. Wie erwähnt, erfolgt dann vorzugsweise während des Motorlaufs keine Berührungsdetektion. Nach einem Stopp des Motors des Instruments 2 wird die Schaltmatrix 8 wieder geöffnet und die Berührungsdetektion ist jetzt wieder aktiv. Der aktuelle Berührungszustand wird detektiert und an die Steuereinheit 7 weitergeleitet.

Das erfindungsgemäße Verfahren zum Betreiben des Systems 1 umfasst daher die folgenden Schritte (a) Überwachen eines Berührens der Instrumente 2, 2' und (b) Initialisieren der Versorgungseinheit 3 für einen Betrieb des Instruments, also beispielsweise zur Verfügung stellen der Ansteuer- oder Parametrieroptionen bzw. Medien für eines der Instrumente 2, abhängig von einer erkannten Berührung des einen Instruments 2.

Insbesondere kann die Gestaltung derart sein, dass alle relevanten Parameter bzw. Parametrieroptionen auf dem Display 6 angezeigt und zum Editieren angeboten werden, sobald der Benutzer das Instrument 2 berührt. Dadurch ist eine "touchähnliche" Bedienung für das Instrument 2 ermöglicht, ohne dass - wie beim eingangs genannten Stand der Technik der Fall - aufwändig durch ein Menü geklickt werden muss oder ein Vorwahlschalter betätigt werden muss. Somit ist eine besonders komfortable Bedienung ermöglicht.

Das System 1 umfasst zwei oder noch mehr entsprechende Instrumente 2, 2'. Im Allgemeinen kann das System 1 eine beliebige Anzahl von entsprechenden Instrumenten 2, 2' aufweisen.

Mit den Mitteln 4 zum Erkennen wird automatisch erkannt, welches der Instrumente 2, 2' berührt worden ist, wobei das System 1 daraufhin für das berührte Instrument 2 entsprechende Ansteuer- oder Parametrieroptionen bzw. die besagten Medien zur Verfügung stellt.

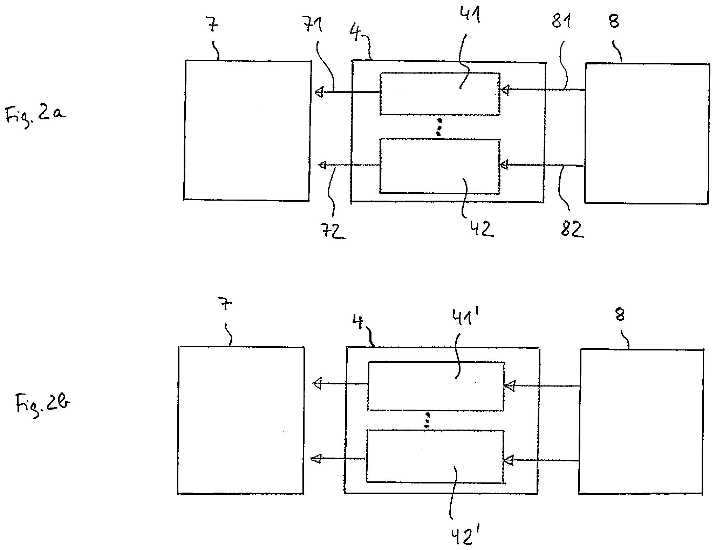

In Fig. 2a ist eine Skizze zu einer möglichen Gestaltung der Mittel 4 zum Erkennen des Berührens gezeigt. Gezeigt ist ein Fall, in dem n Instrumente 2, 2' vorgesehen sind und für jedes der n Instrumente 2, 2' eine Erkennungseinheit 41, 42 in den Mitteln 4 ausgebildet ist, also insgesamt n Erkennungseinheiten 41, 42, von einer ersten Erkennungseinheit 41 bis zu einer n-ten Erkennungseinheit 42 gebildet sind. Dabei ist zwischen jeder der n Erkennungseinheiten 41, 42 einerseits und der Schaltmatrix 8 andererseits jeweils eine Signalverbindungsleitung 81, 82 gebildet. Die Erkennungseinheiten 41, 42 sind hier als kapazitiv wirkend gestaltet. Insbesondere können also die n Erkennungseinheiten 41, 42 als n kapazitive Sensoren gestaltet sein. Von jeder der Erkennungseinheiten 41, 42 ist jeweils eine weitere Signalverbindung 71, 72 zu der Steuereinheit 7 gebildet.

In Fig. 2b ist eine alternative Gestaltung gezeigt. Der Unterschied zu der Gestaltung gemäß Fig. 2a besteht hierbei lediglich darin, dass die Erkennungseinheiten, hier als 41', 42' bezeichnet, als Brumm-Erkennungseinheiten ausgebildet sind.

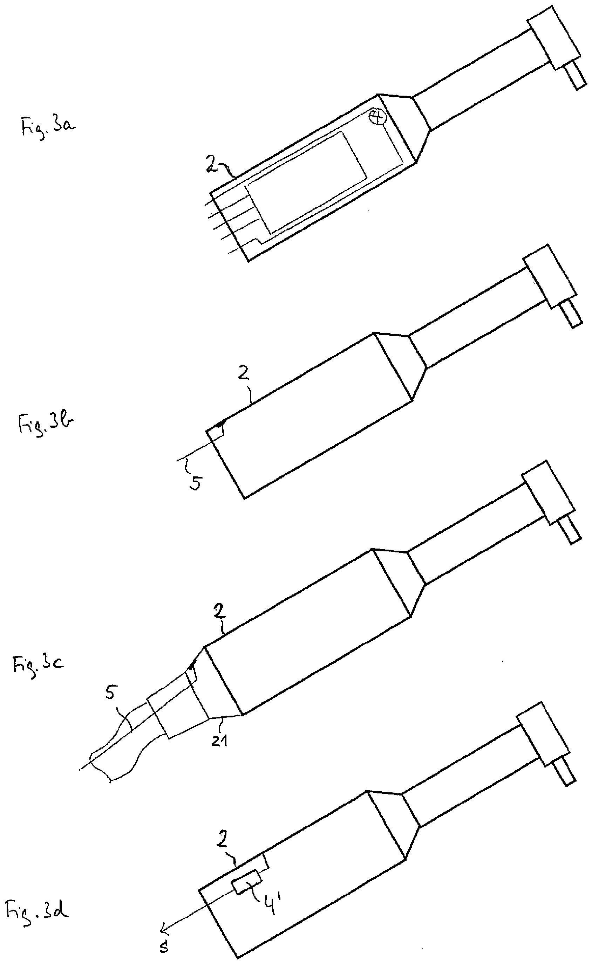

In Fig. 3a ist eine Skizze zu einer möglichen Ausführungsform des Instruments 2 gezeigt. Die Sensorleitung ist hier eine Motorphase oder eine Lichtansteuerleitung oder eine sonstige vorhandene Leitung. Bei der in Fig. 3b gezeigten Ausführung ist eine explizite bzw. eigens für die Erkennung konzipierte Sensorleitung vorgesehen. Das Gehäuse des Instruments 2 ist leitfähig gestaltet oder es ist im Fall eines Kunststoffgehäuses eine kapazitive Ankopplung an die Sensorleitung gebildet.

Bei der in Fig. 3c skizzierten Ausführung ist ist eine Sensorleitung gebildet, die in einem Anschlussstück 21 des Instruments 2 endet. Das Gehäuse des Instruments 2 und das Anschlussstück sind leitfähig gestaltet oder es ist im Fall eines Kunststoffgehäuses eine kapazitive Ankopplung gebildet.

Bei der in Fig. 3d gezeigten Ausführung sind die Mittel 4 zum Erkennen bzw. ist eine entsprechende Erkennungseinheit 4', also beispielsweise ein kapazitiver Sensor 4', in das Instrument 2 integriert gebildet. Von dieser Erkennungseinheit 4' wird bei einer Berührung des Instruments 2 ein Signal S zur Steuereinheit 7 gesendet.

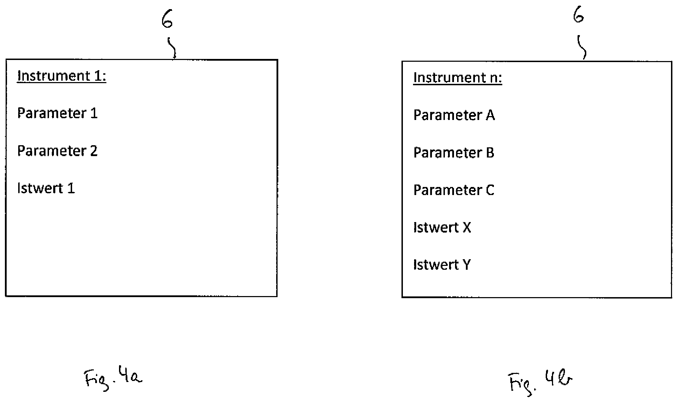

In den Figuren 4a und 4b sind Skizzen zu einer möglichen Darstellung auf dem Display 6 gezeigt, Fig. 4a im Fall der Auswahl des ersten Instruments und Fig. 4b im Fall der Auswahl des n-ten Instruments.

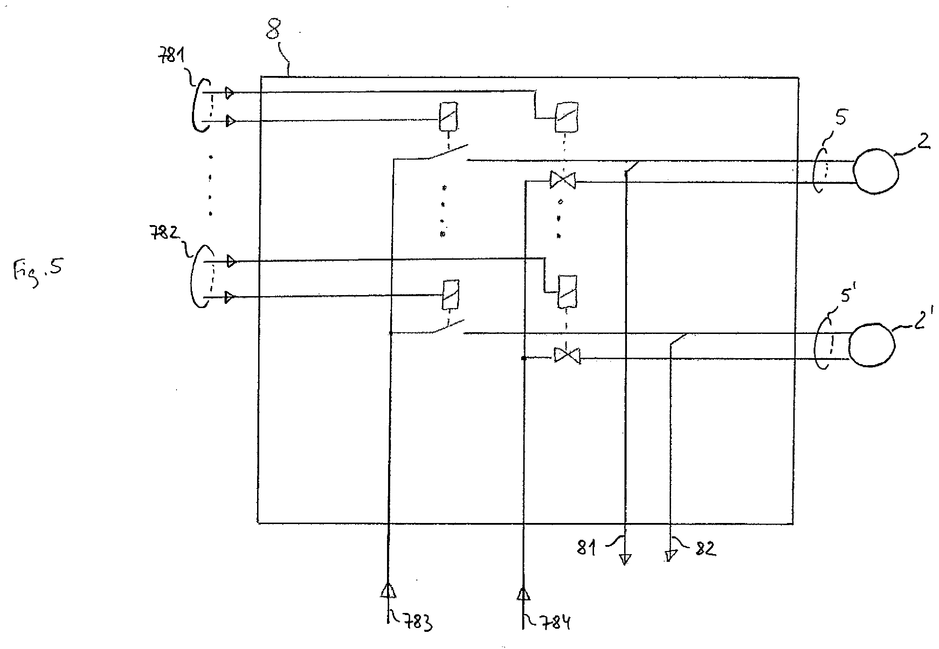

Fig. 5 zeigt eine Skizze zu einer möglichen Ausführung der Schaltmatrix 8. Zwischen jedem der n Instrumente 2, 2' einerseits und der Schaltmatrix 8 andererseits ist - wie weiter oben bereits beschrieben - jeweils eine Versorgungsleitung 5, 5' vorgesehen. Im Fall einer Berührung des ersten Instruments 2 wird ein entsprechendes Signal von der Schaltmatrix 8 über die Signalverbindungsleitung 81 zu den Mitteln 4 zum Erkennen des Berührens gesendet, im Fall einer Berührung des n-ten Instruments 2' ein entsprechendes Signal über die Signalverbindungsleitung 82. Wenn eine entsprechende Erkennungseinheit in dem betreffenden Instrument integriert ist wird über die entsprechende Leitung 81 bzw. 82 direkt ein Touchsignal gesendet.

Weitere n Leitungen 781, 782 führen von der Steuereinheit 7 zur Schaltmatrix 8; wenn das erste Instrument 2 ausgewählt wird, erfolgt eine entsprechende Signalübertragung über die Leitung 781, wenn das n-te Instrument 2' ausgewählt wird, eine entsprechende Signalübertragung über die n-te Leitung 782. Über eine noch weitere Leitung 783 werden Ansteuersignale "Licht", "Motor" etc. zur Schaltmatrix 8 gesendet. Schließlich dient eine Medienleitung 784 zur Zufuhr von Luft bzw. Wasser.

## Patentansprüche

1. Zahnärztliches Behandlungs- oder Untersuchungssystem (1) mit mehreren Behandlungs- oder Untersuchungsinstrumenten (2, 2') sowie einer mit den mehreren Behandlungs- oder Untersuchinstrumenten (2, 2') gekoppelten Versorgungseinheit (3), **dadurch gekennzeichnet,**
**dass** das System (1) Mittel (4) zum Erkennen des Berührens eines ausgewählten Instruments (2) von den mehreren Behandlungs- oder Untersuchungsinstrumenten (2, 2') aufweist und dazu ausgebildet ist, abhängig von einer erkannten Berührung des ausgewählten Behandlungs- oder Untersuchungsinstruments (2) die Versorgungseinheit (3) für einen Betrieb des ausgewählten Behandlungs- oder Untersuchungsinstruments (2) zu initialisieren,
wobei die Mittel (4) zum Erkennen des Berührens mindestens einen kapazitiven Sensor umfassen,
wobei ein Erkennen des Berührens des Behandlungs- oder Untersuchungsinstruments (2) lediglich in einem nicht aktivierten Zustand der zur Auswahl stehenden Instrumente (2) durchgeführt wird,
wobei die Versorgungseinheit (3) ein Display (6) aufweist oder mit einem Display verbunden ist, auf dem eine Darstellung von Betriebsparametern des ausgewählten Instruments (2) erfolgt, wobei die Darstellung abhängig von dem Erkennen des Berührens des ausgewählten Instruments (2) erfolgt.

2. Zahnärztliches Behandlungs- oder Untersuchungssystem nach Anspruch 1,
**dadurch gekennzeichnet,**
**dass** das Initialisieren der Versorgungseinheit (3) das zur Verfügung stellen von Ansteuer- oder Parametrieroptionen für das ausgewählte Behandlungs- oder Untersuchungsinstrument (2) und/oder von Medien, z.B. von Luft oder Wasser, umfasst.

3. Zahnärztliches Behandlungs- oder Untersuchungssystem nach Anspruch 1 oder 2, **dadurch gekennzeichnet,**
**dass** das Initialisieren der Versorgungseinheit (3) das elektrische Verbinden des ausgewählten Behandlungs- oder Untersuchungsinstruments (2) mit einem Leistungsausgang einer Steuereinheit (7) umfasst.

4. Zahnärztliches Behandlungs- oder Untersuchungssystem nach einem der vorherigen Ansprüche,
**dadurch gekennzeichnet,**
**dass** die Mittel (4) zum Erkennen des Berührens die kapazitive Einkopplung auf das Gehäuse des ausgewählten Instruments (2) oder auf Leitungen davon bewerten, wobei die kapazitive Einkopplung insbesondere durch Messen von Brumm- oder Störspannungen ermittelt wird.

5. Zahnärztliches Behandlungs- oder Untersuchungssystem nach einem der Ansprüche 1 bis 3,
**dadurch gekennzeichnet,**
**dass** die Mittel (4) zum Erkennen des Berührens eine Kapazitätsänderung des ausgewählten Instruments (2) bewerten.

6. Zahnärztliches Behandlungs- oder Untersuchungssystem nach einem der vorherigen Ansprüche,
**dadurch gekennzeichnet,**
**dass** die Mittel (4) zum Erkennen des Berührens eine in der Versorgungseinheit (3) angeordnete Bewertungseinheit umfassen, welche über eine Sensorleitung (5) mit dem ausgewählten Instrument (2) verbunden ist.

7. Zahnärztliches Behandlungs- oder Untersuchungssystem nach Anspruch 6,
**dadurch gekennzeichnet,**
**dass** die Sensorleitung (5) durch eine Stromversorgungsleitung für das ausgewählte Instrument (2) gebildet ist.

8. Zahnärztliches Behandlungs- oder Untersuchungssystem nach einem der vorhergehenden Ansprüche,
**dadurch gekennzeichnet,**
**dass** die Mittel (4) zum Erkennen des Berührens in das ausgewählte Behandlungs- oder Untersuchungsinstrument (2) integriert sind.

9. Verfahren zum Betreiben eines zahnärztlichen Behandlungs- oder Untersuchungssystems (1) mit mehreren Behandlungs- oder Untersuchungsinstrumenten (2, 2') sowie einer mit den mehreren Behandlungs- oder Untersuchungsinstrumenten (2, 2') gekoppelten Versorgungseinheit (3),
**dadurch gekennzeichnet,**
**dass** ein Berühren der Behandlungs- oder Untersuchungsinstrumente (2, 2') überwacht wird und abhängig von einer erkannten Berührung eines der Behandlungs- oder Untersuchungsinstrumente (2) die Versorgungseinheit (3) für einen Betrieb des ausgewählten Behandlungs- oder Untersuchungsinstruments (2) initialisiert wird, wobei zum Erkennen des Berührens des einen der Behandlungs- oder Untersuchungsinstrumente (2) mindestens ein kapazitiver Sensor verwendet wird, wobei ein Erkennen des Berührens des einen der Behandlungs- oder Untersuchungsinstrumente (2) lediglich in einem nicht aktivierten Zustand des einen der Behandlungs- oder Untersuchungsinstrumente (2) durchgeführt wird,
wobei die Versorgungseinheit (3) ein Display (6) aufweist oder mit einem Display verbunden ist, auf dem eine Darstellung von Betriebsparametern des einen der Behandlungs- oder Untersuchungsinstrumente (2) erfolgt, wobei die Darstellung abhängig von dem Erkennen des Berührens des einen der Behandlungs- oder Untersuchungsinstrumente (2) erfolgt.

10. Verfahren nach Anspruch 9,
**dadurch gekennzeichnet,**
**dass** das Initialisieren der Versorgungseinheit (3) das zur Verfügung stellen von Ansteuer- oder Parametrieroptionen für das ausgewählte Behandlungs- oder Untersuchungsinstrument (2) und/oder von Medien, z.B. von Luft oder Wasser, umfasst.

11. Verfahren nach Anspruch 9 oder 10,
**dadurch gekennzeichnet,**
**dass** das Initialisieren der Versorgungseinheit (3) das elektrische Verbinden des ausgewählten Behandlungs- oder Untersuchungsinstruments (2) mit einem Leistungsausgang einer Steuereinheit (7) umfasst.

12. Verfahren nach einem der Ansprüche 9 bis 11,
**dadurch gekennzeichnet,**
**dass** zum Erkennen des Berührens die kapazitive Einkopplung auf das Gehäuse des einen der Behandlungs- oder Untersuchungsinstrumente (2) oder auf Leitungen davon bewertet werden,
wobei die kapazitive Einkopplung insbesondere durch Messen von Brumm- oder Störspannungen ermittelt wird.

13. Verfahren nach einem der Ansprüche 9 bis 11,
**dadurch gekennzeichnet,**
**dass** zum Erkennen des Berührens eine Kapazitätsänderung des einen der Behandlungs- oder Untersuchungsinstrumente (2) bewertet wird.

## Claims

1. Dental treatment or examination system (1) having a plurality of treatment or examination instruments (2, 2') and a supply unit (3) coupled to the plurality of treatment or examination instruments (2, 2'),
**characterized**
**in that** the system (1) has means (4) for identifying touching of a selected instrument (2) from the plurality of treatment or examination instruments (2, 2') and said system is embodied to initialize the supply unit (3) for an operation of the selected treatment or examination instrument (2) depending on an identification of touching of the selected treatment or examination instrument (2), wherein the means (4) for identifying touching comprise at least one capacitive sensor,
wherein an identification of touching of the treatment or examination instrument (2) is only carried out in a non-activated state of the instruments (2) available for selection,
wherein the supply unit (3) has a display (6) or is linked to a display, on which a representation of operating parameters of the selected instrument (2) is effectuated, wherein the representation is effectuated depending on the identification of touching of the selected instrument (2).

2. Dental treatment or examination system according to Claim 1,
**characterized**
**in that** the initialization of the supply unit (3) comprises the provision of actuation or parameterization options for the selected treatment or examination instrument (2) and/or the provision of media, e.g. of the air or water.

3. Dental treatment or examination system according to Claim 1 or 2,
**characterized**
**in that** the initialization of the supply unit (3) comprises electrically connecting the selected treatment or examination instrument (2) to a power output of the control unit (7).

4. Dental treatment or examination system according to any one of the preceding claims,
**characterized**
**in that** the means (4) for identifying touching evaluate the capacitive coupling on the housing of the selected instrument (2) or on lines thereof,
wherein the capacitive coupling is ascertained, in particular, by measuring ripple or interference voltages.

5. Dental treatment or examination system according to any one of Claims 1 to 3,
**characterized**
**in that** the means (4) for identifying touching evaluate a change in capacitance of the selected instrument (2).

6. Dental treatment or examination system according to any one of the preceding claims,
**characterized**
**in that** the means (4) for identifying touching comprise an evaluation unit arranged in the supply unit (3), said evaluation unit being connected to the selected instrument (2) by a sensor line (5).

7. Dental treatment or examination system according to Claim 6,
**characterized**
**in that** the sensor line (5) is formed by a power supply line for the selected instrument (2).

8. Dental treatment or examination system according to any one of the preceding claims,
**characterized**
**in that** the means (4) for identifying touching are integrated into the selected treatment or examination instrument (2).

9. Method for operating a dental treatment or examination system (1) having a plurality of treatment or examination instruments (2, 2') and a supply unit (3) coupled to the plurality of treatment or examination instruments (2, 2'),
**characterized**
**in that** touching of the treatment or examination instruments (2, 2') is monitored and the supply unit (3) is initialized for an operation of the selected treatment or examination instrument (2) depending on an identification of touching of one of the treatment or examination instruments (2),
wherein at least one capacitive sensor is used for identifying touching of the one of the treatment or examination instruments (2),
wherein an identification of touching of the one of the treatment or examination instruments (2) is only effectuated in a non-activated state of the one of the treatment or examination instruments (2),
wherein the supply unit (3) has a display (6) or is connected to a display, on which a representation of operating parameters of the one of the treatment or examination instruments (2) is effectuated, wherein the representation is effectuated depending on the identification of touching of the one of the treatment or examination instruments (2).

10. Method according to Claim 9,
**characterized**
**in that** the initialization of the supply unit (3) comprises the provision of actuation or parameterization options for the selected treatment or examination instrument (2) and/or the provision of media, e.g. of the air or water.

11. Method according to Claim 9 or 10,
**characterized**
**in that** the initialization of the supply unit (3) comprises electrically connecting the selected treatment or examination instrument (2) to a power output of a control unit (7).

12. Method according to any one of Claims 9 to 11,
**characterized**
**in that** the capacitive coupling on the housing of the one of the treatment or examination instruments (2) or lines thereof is evaluated for identifying touching,
wherein the capacitive coupling is ascertained, in particular, by measuring ripple or interference voltages.

13. Method according to any one of Claims 9 to 11,
**characterized**
**in that** a change in capacitance of the one of the treatment or examination instruments (2) is evaluated for identifying touching.

## Revendications

1. Système d'analyse ou de traitement dentaire (1) comportant plusieurs instruments d'analyse ou de traitement (2, 2') ainsi qu'une unité d'alimentation (3) couplée aux plusieurs instruments d'analyse ou de traitement (2, 2'),
**caractérisé en ce que**
le système (1) présente des moyens (4) pour reconnaître le contact d'un instrument sélectionné (2) parmi les plusieurs instruments d'analyse ou de traitement (2, 2') et il est configuré pour initialiser, en fonction d'un contact reconnu de l'instrument d'analyse ou de traitement sélectionné (2), l'unité d'alimentation (3) pour un fonctionnement de l'instrument d'analyse ou de traitement sélectionné (2),
dans lequel les moyens (4) pour reconnaître le contact comprennent au moins un détecteur capacitif,
dans lequel on effectue une reconnaissance du contact de l'instrument d'analyse ou de traitement (2) uniquement dans un état non activé des instruments sélectionnables (2),
dans lequel l'unité d'alimentation (3) présente un écran d'affichage (6) ou est reliée à un écran d'affichage, sur lequel on réalise une représentation de paramètres de fonctionnement de l'instrument sélectionné (2), dans lequel on réalise la représentation en fonction de la reconnaissance du contact de l'instrument sélectionné (2).

2. Système d'analyse ou de traitement dentaire selon la revendication 1, **caractérisé en ce que** l'initialisation de l'unité d'alimentation (3) comprend la mise à disposition d'options de commande ou de paramétrage pour l'instrument d'analyse ou de traitement sélectionné (2) et/ou de fluides, par exemple d'air ou d'eau.

3. Système d'analyse ou de traitement dentaire selon la revendication 1 ou 2, **caractérisé en ce que** l'initialisation de l'unité d'alimentation (3) comprend la connexion électrique de l'instrument d'analyse ou de traitement sélectionné (2) à une sortie de puissance d'une unité de commande (7).

4. Système d'analyse ou de traitement dentaire selon l'une quelconque des revendications précédentes, **caractérisé en ce que** les moyens (4) pour reconnaître le contact exploitent le couplage capacitif sur le boîtier de l'instrument sélectionné (2) ou sur des conducteurs de celui-ci, dans lequel on détermine le couplage capacitif en particulier par la mesure de tensions d'ondulation ou de tensions parasites.

5. Système d'analyse ou de traitement dentaire selon l'une quelconque des revendications 1 à 3, **caractérisé en ce que** les moyens (4) pour reconnaître le contact exploitent une variation de capacité de l'instrument sélectionné (2).

6. Système d'analyse ou de traitement dentaire selon l'une quelconque des revendications précédentes, **caractérisé en ce que** les moyens (4) pour reconnaître le contact comprennent une unité d'exploitation disposée dans l'unité d'alimentation (3), qui est reliée par une ligne de détecteur (5) à l'instrument sélectionné (2).

7. Système d'analyse ou de traitement dentaire selon la revendication 6, **caractérisé en ce que** la ligne de détecteur (5) est formée par une ligne d'alimentation électrique pour l'instrument sélectionné (2).

8. Système d'analyse ou de traitement dentaire selon l'une quelconque des revendications précédentes, **caractérisé en ce que** les moyens (4) pour reconnaître le contact sont intégrés dans l'instrument d'analyse ou de traitement sélectionné (2).

9. Procédé de fonctionnement d'un système d'analyse ou de traitement dentaire (1) comportant plusieurs instruments d'analyse ou de traitement (2, 2') ainsi qu'une unité d'alimentation (3) couplée aux plusieurs instruments d'analyse ou de traitement (2, 2'),
**caractérisé en ce que**
on surveille un contact des instruments d'analyse ou de traitement (2, 2') et on initialise, en fonction d'un contact reconnu d'un des instruments d'analyse ou de traitement (2), l'unité d'alimentation (3) pour un fonctionnement de l'instrument d'analyse ou de traitement sélectionné (2),
dans lequel on utilise au moins un détecteur capacitif pour reconnaître le contact de cet instrument d'analyse ou de traitement (2),
dans lequel on effectue une reconnaissance du contact de cet instrument d'analyse ou de traitement (2) uniquement dans un état non activé de cet instrument d'analyse ou de traitement (2),
dans lequel l'unité d'alimentation (3) présente un écran d'affichage (6) ou est reliée à un écran d'affichage, sur lequel on réalise une représentation de paramètres de fonctionnement de cet instrument d'analyse ou de traitement (2), dans lequel on réalise la représentation en fonction de la reconnaissance du contact de cet instrument d'analyse ou de traitement (2).

10. Procédé selon la revendication 9, **caractérisé en ce que** l'initialisation de l'unité d'alimentation (3) comprend la mise à disposition d'options de commande ou de paramétrage pour l'instrument d'analyse ou de traitement sélectionné (2) et/ou de fluides, par exemple d'air ou d'eau.

11. Procédé selon la revendication 9 ou 10, **caractérisé en ce que** l'initialisation de l'unité d'alimentation (3) comprend la connexion électrique de l'instrument d'analyse ou de traitement sélectionné (2) à une sortie de puissance d'une unité de commande (7).

12. Procédé selon l'une quelconque des revendications 9 à 11, **caractérisé en ce que** l'on exploite pour reconnaître le contact le couplage capacitif sur le boîtier de cet instrument d'analyse ou de traitement (2) ou sur des conducteurs de celui-ci, dans lequel on détermine le couplage capacitif en particulier par la mesure de tensions d'ondulation ou de tensions parasites.

13. Procédé selon l'une quelconque des revendications 9 à 11, **caractérisé en ce que** l'on exploite pour reconnaître le contact une variation de capacité de cet instrument d'analyse ou de traitement (2).
